# EUROPEAN PATENT APPLICATION

(11) **EP 4 151 208 A1**
(43) Date of publication of application: **22.03.2023**
(21) Application number: 21197506.5
(22) Date of filing: 17.09.2021
(51) Int. Cl.: A61K 31/137, A61K 31/366, A61K 31/40, A61K 31/4439, A61K 31/496, A61K 31/5375, A61K 31/5377, A61K 33/243, A61K 45/06, A61P 35/00, A61P 35/04

(54) **NOVEL SYNERGISTIC COMBINATIONS AND METHODS OF USES THEREOF FOR TREATING CANCERS**

(71) Applicant: UNIVERSITE DE BORDEAUX, 33000 Bordeaux (FR); INSTITUT NATIONAL DE LA SANTE ET DE LA RECHERCHE MEDICALE - INSERM, 75013 Paris (FR)
(72) Inventor: GROSSET, Christophe, 33600 PESSAC (FR); HAGEDORN, Martin, 33600 PESSAC (FR); RAHAL, Farah, 33000 BORDEAUX (FR); KHOUBAI, Fatma Zohra, 33300 BORDEAUX (FR)
(74) Representative: Lecca, Patricia S.

(57) **Abstract**

The invention relates to novel compositions, combinations and methods relating to compounds which inhibit EZH2 and their uses for treating and/or preventing tumors associated with methyltransferase EZH2. More specifically the invention relates to synergistic bi-therapy compositions for use in a method of treating and/or preventing tumors associated with methyltransferase EZH2, comprising administering to a subject in need, a therapeutically effective amount of the composition, wherein said composition comprises the combination of EZH2 inhibitor and one HMG-CoA reductase inhibitor or statin. The present invention also relates to synergistic tri-therapy compositions as well as methods of use thereof for treating and/or preventing specific types of cancers and tumors comprising further administering an effective amount of one or more anticancer drugs or chemotherapeutic agents in combination with the above bi-therapy compositions.

## Description

### FIELD OF INVENTION

The invention relates to novel synergistic combinations as well as methods of use for treating and/or preventing specific types of cancers and tumors, such as in particular cancers and tumors associated with histone methyltransferase EZH2. The present invention also relates to pharmaceutical compositions comprising said synergistic combinations for use in the treatment and/or the prevention of histone methyltransferase EZH2-associated cancers and/or tumors.

### BACKGROUND OF THE INVENTION

Epigenetic modification could regulate chromatin state and gene expression through DNA methylation and demethylation, histone modification, chromatin remodeling etc..., without altering DNA sequences. Polycomb group proteins (PcGs), a group of important epigenetic regulators, play an important part in cell proliferation and are critical factors of pluripotency and differentiation of stem cells as well as aberrant gene expression during the malignant transformation. PcG proteins contain two core complexes, respectively are the maintenance complex polycomb repressive complex 1 (PRC1) and the initiation complex polycomb repressive complex 2 (PRC2). PRC1 has been known to mono-ubiquitinate the histone H2A at Lys 119 through RING1A and RING1B ubiquitin ligases. PRC2 has been considered to catalyze the mono-, di-, and tri-methylation of histone H3 at Lys 27 to regulate gene transcription.

Enhancer of zeste homolog 2 (EZH2) is an evolutionary conserved gene identified in many species, sharing similar structural motifs and domains. The histone methyltransferase EZH2 has been identified as a catalytic subunit of PRC2 for tri-methylation of histone H3 at Lys 27 (H3K27me3) by SET domain in its C-terminus, which silences targeted genes and is involved in various biological functions (*e.g*., cell cycle, cell proliferation, cell differentiation, etc...). The role of EZH2 in cancer progression has also been considered. EZH2 is aberrantly overexpressed in various malignant tumors, such as prostate cancer, breast cancer, and ovarian cancer. EZH2 mediates H3K27me3 and functions as a critical factor in promoting tumor growth and metastasis in many malignant tumor models. In addition, EZH2 gain or loss of function mutations have also been discovered in various cancer. Some studies indicate that inhibition of EZH2 by small molecular inhibitors or gene knockdown results in reduced cancer cell growth and tumor formation. Beyond playing its role in a PCR2-dependent manner as a histone modifier, EZH2 also acts in a PCR2 and histone independent manner in cancer. For example, EZH2 can methylate non-histone protein STAT3 in glioblastoma, and participate in androgen receptor-associated complexes in castration-resistant prostate cancer (CRPC) as a co-activator. As described by Gan L et al., (Biomark Res 6, 10 (2018). https: doi.org/10.1186/s40364-018-0122-2*),* the diverse functions of EZH2 in cancer derive from its genetic, transcriptional, post-transcriptional and post-translational regulation in different circumstances and different types of cancer.

Further findings to support an oncogenic role for EZH2 have more recently emerged. Studies have shown that recurrent heterozygous point mutations at tyrosine 641 (Y641) within the C-terminal catalytic SET domain of EZH2 occur in 22% of germinal center B-cell (GCB) diffuse large cell B-cell lymphomas (DLBCL) and in 7% to 12% of follicular lymphomas (FL). This was shown to confer gain-of-function of enzyme activity resulting in augmented conversion of H3K27 to the trimethylated form. Y641 mutants (Y641F, Y641N, Y641S, Y641C, and Y641H) have reduced methylation activity of unmethylated H3K27 but enhanced activity for the dimethylated version of H3k27. The mutant thus cooperates together with wild-type EZH2 to shift the steady state of H3K27 to favor trimethylation and thus represses expression of Polycomb targets. EZH2 point mutations at the A677 and A687 residues have also been identified in non-Hodgkin lymphomas (NHL), where they similarly result in hypertrimethylation of H3K27. Additional support for a gain-of-function role for mutant EZH2 in cancer comes from the identification of cancer-associated loss-of-function mutations in other chromatin regulators that normally antagonize EZH2 activity. UTX (ubiquitously transcribed tetratricopeptide repeat gene on X chromosome) is a histone demethylase that functions in part by antagonizing EZH2 activity by removing methyl groups from di- and trimethylated H3K27. Inactivating mutations affecting UTX occur in several types of human cancer, including multiple myeloma, medulloblastoma, esophageal cancer, bladder cancer, pancreatic cancer, and renal cancers. These mutations include homozygous (in females) or hemizygous (in males) large deletions, nonsense mutations, small frame-shifting insertion/deletions, and consensus splice site mutations that lead to a premature termination codon. Almost all mutations are predicted to result in loss of the JmjC domain of UTX, which is essential for its demethylase activity, and have been shown to cause increased levels of H3K27 trimethylation. Therefore, as described by Kim KH et al. (Targeting EZH2 in cancer. Nat Med. 2016;22(2):128-134. doi:10.1038/nm.4036*),* loss-of-function mutations in UTX may be analogous to those caused by gain-of-function mutations in EZH2.

In spite of this understanding, the efficacy of EZH2 inhibitors for preventing and/or treating tumors and cancer has remained controversial. Several candidates have been or are currently in clinical trials and the data available thus far is not conclusive.

The first EZH2 inhibitor was 3-deazaneplanocin A (DZNep). DZNep, a known S-adenosyl-L-homocysteine (SAH) hydrolase inhibitor, indirectly inhibits EZH2 through the increase of SAH, which directly represses S-adenosyl-L-methionine-dependent histone methyltransferase activity. DZNep globally inhibits histone methylation but is not specific to EZH2.

A second candidate GSK126 - also known as GSK2816126 - was shown to inhibit wild type and Y641 mutant EZH2 with similar potency and is highly selective compared to EZH1 (150-fold increased potency) or 20 other methyltransferases (> 1000-fold selective for EZH2). A multicentric phase 1 clinical trial was initiated back in 2019 to evaluate the safety, maximum-tolerated dose (MTD), pharmacokinetics, and pharmacodynamics of GSK126 (NCT02082977) in patients with relapsed/refractory solid tumors and hematologic malignancies. Forty-one participants (21 solid tumors, 20 lymphomas) received escalating doses of GSK2816126 ranged from 50 to 3000 mg twice weekly as an intravenous solution for 28 days (3 weeks on/1 week off). This clinical phase was however terminated since the results showed insufficient evidence of clinical activity and did not justify further clinical investigation while the dosing method and relatively short half-life limited effective exposure (See, https://clinicaltrials.gov/ct2/show/NCT02082977). The results of this clinical phase were also reported by Yap TA et al. (Clin Cancer Res. 2019 Dec 15;25(24):7331-7339) concluded that GSK2816126 was not a viable drug to target EZH2 in patients with refractory/relapsed solid and hematologic malignancies, despite preclinical data showing sensitivity of multiple solid tumor and lymphoma cell lines. The study defined the maximum tolerated dose (MTD) of GSK2816126 as 2,400 mg, but at this level, the drug showed inadequate clinical activity, with off-target dose-limiting toxicity (DLT) precluding further dose escalation.

Several other S-adenosyl-methionine-competitive inhibitors of EZH2 were also developed including *inter alia* GSK343, GSK926 and tazemetostat (E7438/EPZ6438). GSK926 and GSK343 can suppress histone H3K27me3 level and inhibit EZH2 activity in breast and prostate cancer cells, while GSK343 can only be used *in vitro* due to the high clearance in rat pharmacokinetic studies.

Tazemetostat showed improved potency and pharmacokinetic properties and can be administered orally. Wiese M et al. (Klin Padiatr. 2016 Apr;228(3):113-7*)* investigated the correlation of expression of EZH2 and other PRC2 genes (EZH1, SUZ12, EED) with overall survival of pediatric glioblastoma multiform (GBM) patients and the cytotoxic impact of EZH2 inhibition by tazemetostat in pediatric glioblastoma multiform/diffuse intrinsic pontine glioma/diffuse midline glioma (GBM/DIPG/DMG) cells harboring either a H3.3 mutation or a H3 wildtype. Wiese et al. however concluded that the treatment with this EZH2 inhibitor does not represent a promising approach in these tumors. Still the USFDA granted on June 2020 an accelerated approval to the EZH2 inhibitor tazemetostat (TAZVERIK, Epizyme, Inc.) for adult patients with relapsed or refractory (R/R) follicular lymphoma (FL) whose tumors are positive for an EZH2 mutation as detected by an FDA-approved test and who have received at least 2 prior systemic therapies, as well as for adult patients with R/R FL who have no satisfactory alternative treatment options.

As such, there is currently a need for new methods and compositions with greater therapeutic efficacy for treating and/or preventing the numerous malignancies associated with methyltransferase EZH2 activity. The present invention addresses this need and provides novel therapies.

### SUMMARY OF THE INVENTION

The present invention relates to synergistic bi-therapy compositions as well as methods of use thereof for treating and/or preventing specific types of cancers and tumors comprising administering to a subject in need a therapeutically effective amount of the composition, wherein said composition comprises the combination of EZH2 inhibitors and one statin.

The present invention also relates to synergistic tri-therapy compositions as well as methods of use thereof for treating and/or preventing specific types of cancers and tumors comprising further administering an effective amount of one or more anticancer drugs or chemotherapeutic agents in combination with the above bi-therapy compositions.

Synergistic bi-therapy and tri-therapy compositions according to the present invention are particularly effective in treating specific types of cancer and tumors associated with histone methyltransferase EZH2.

Most importantly, synergistic bi-therapies and tri-therapies according to the present invention allow reducing the dose regimen of EZH2 inhibitors compared to previous clinical trials, and thus exhibit a much-reduced toxicity while retaining therapeutic efficiencies against said tumors. Indeed, Applicant showed herein below that administering the combinations of GSK126 with at least one statin and/or one or more anticancer drugs or chemotherapeutic agents sensitize the cancers or tumors to GSK126, thereby reversing or reducing the resistance of patients to GSK126.

### BRIEF DESCRIPTION OF THE FIGURES

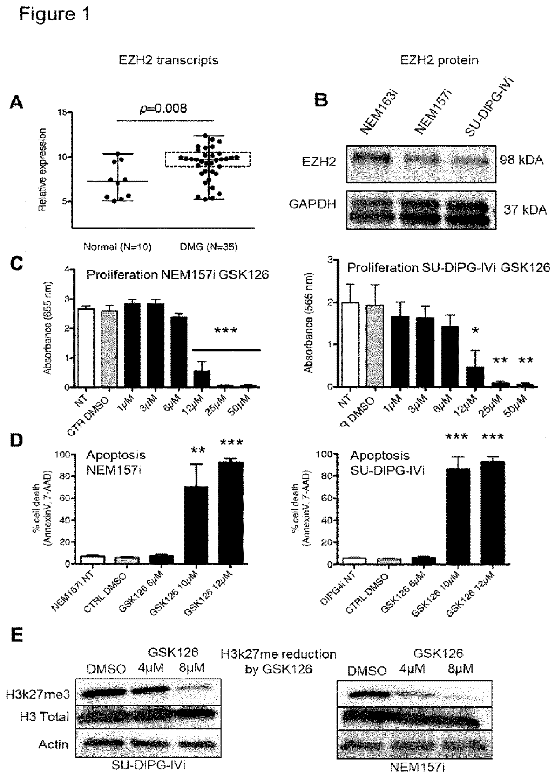

**Figures 1A****-E:** show that DMG cells are sensitive to GSK126 inhibitor at low doses and that GSK126 blocks H3k27me3 trimethylation. (A) EZH2 transcripts are significantly overexpressed in a series of DMG biopsies (n=35) compared to normal brains (n=10), microarray data retrieved from GSE50021. Dashed square delimits samples regrouped around median expression. Parametric t test between DMG biopsies versus normal brains. (B) Patient-derived cell lines express EZH2 protein detected by Western Blot. (C-D) GSK126 strongly inhibits proliferation of NEM157i and SU-DIPG-IVi DMG cells (C) and induces tumor cell death after 5 days of treatment (D). One way ANOVA (n>3, p<0.0001), Bonferroni's multiple comparisons post-test. (E) H3k27me3 trimethylation is reduced in a dose-dependent manner in GSK126 treated NEM157i and SU-DIPG-IVi DMG cells. One blot representative of 3 independent experiments. *, p<0.05; **, p<0.01; ***, p<0.001.

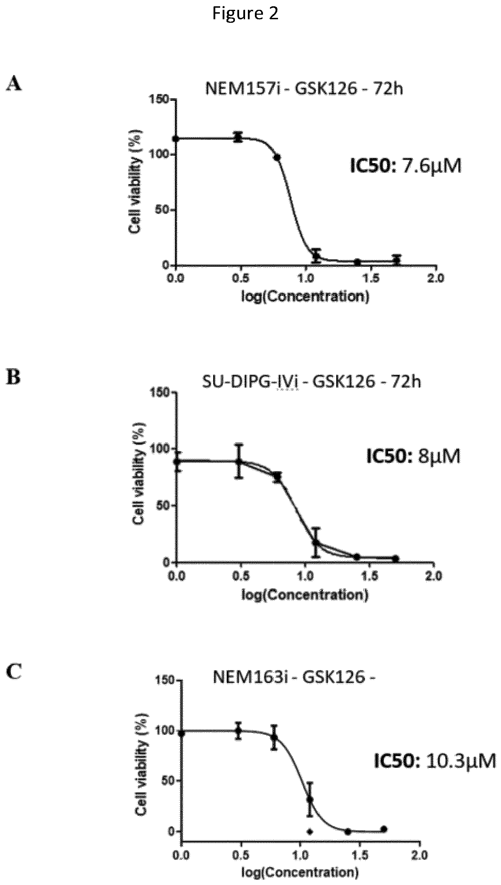

**Figures 2A****-D:** show that DMG cells are sensitive to GSK126 inhibitor at low doses. (A-C) the cell viability of SU-DIPG-IVi (A), NEM157i (B), and NEM163i (C) DMG cells was measured after 72 h of treatment in presence of increasing doses of GSK126 (n=3). For each cell line, IC50 is shown in the corresponding graph.

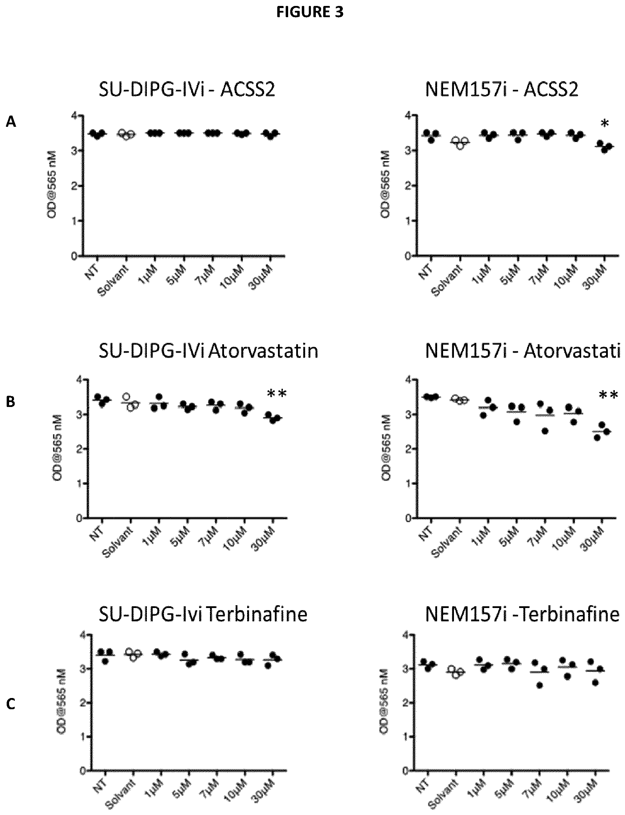

**Figures 3A****-C:** show the absence of effects of inhibitors of cholesterol biosynthesis pathway enzymes (doses shown on X-axis) on the viability of SU-DIPG-IVi and NEM157i DMG cells. (A-C) Three different chemical inhibitors of different enzymes implicated in the cholesterol biosynthesis pathway and induced by GSK126 [(A) Terbinafine: squalene epoxidase - SQLE, (B) Atorvastatin: hydroxymethylglutaryl-coenzyme A (HMG-CoA) reductase - HMGSC1, (C) ACSS2 inhibitor: acetate-dependent acetyl-CoA synthetase 2 - ACCS2] have no effect on DMG cell proliferation up to 30µM, suggesting insensibility of the cells to these inhibitors in the absence of GSK126. One way ANOVA (n=3, p<0.0001), Bonferroni's multiple comparisons post-test. *, p<0.05; **, p<0.01.

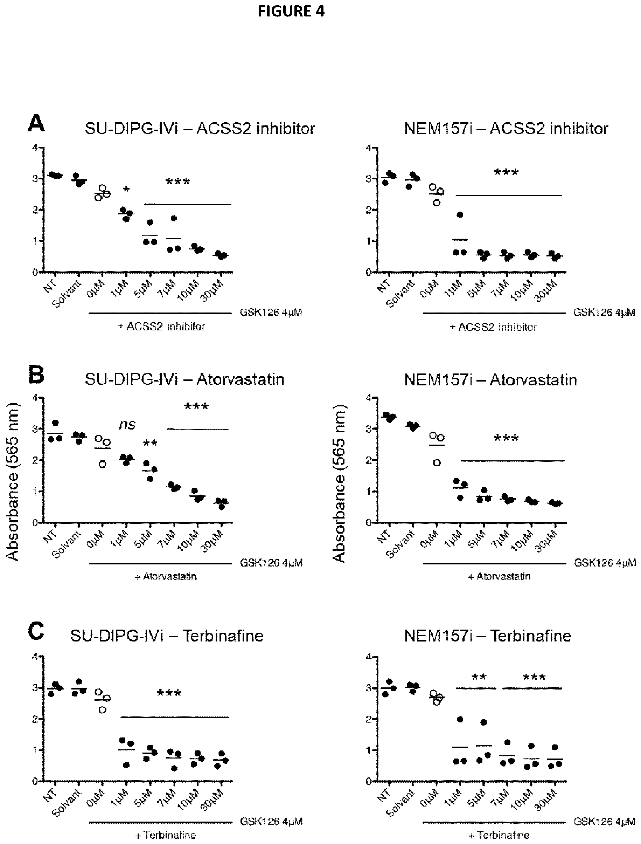

**Figures 4A****-C:** show synergistic effect of the bi-therapy comprising GSK126 and inhibitors of cholesterol biosynthesis pathway enzymes on DMG cells. (A-C) Cell growth assays of NEM157i and SU-DIPG-IVi cells (as indicated) exposed to 4µM of GSK126, a dose which has no growth inhibitory effects on DMG cells (open circles), and to increasing doses of ACSS2 inhibitor (A), Atorvastatin (B) or Terbinafine (C). One way ANOVA (n=3, p<0.0001), Bonferroni's multiple comparisons post-test. Bi-therapy treatment shows significant proliferation inhibition starting at low micro molar doses of statins which show no effects alone (see Figure 3). **, p<0.01; ***, p<0.001.

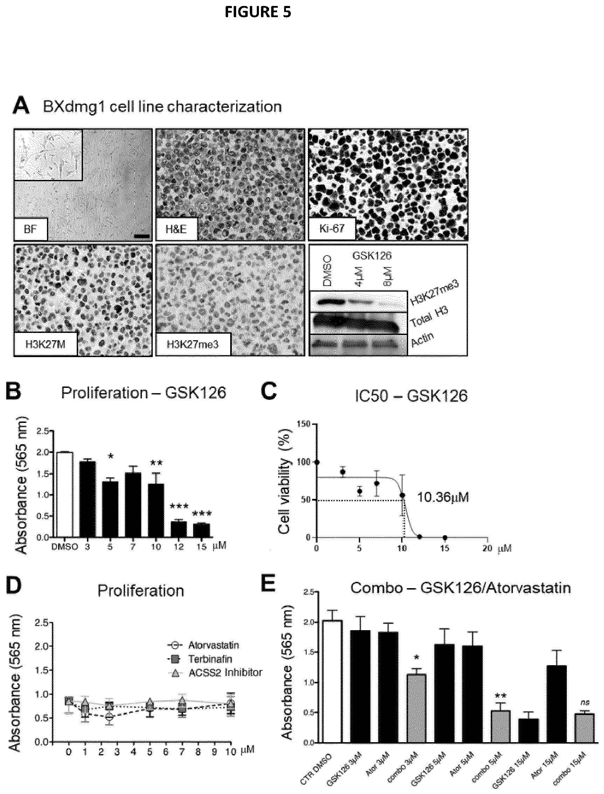

**Figures 5A****-E:** show the molecular and phenotypical characteristics of the new primary DMG cell line BXdmgl. (A) BXdmgl primary cell characterization. Upper left panel: bright field microscopy showing cell morphology of early passage BXdmgl primary cells. Bar = 200µm. Upper middle panel: H&E staining of Cytoblock preparation of cells revealing nuclear irregularities. Upper right panel: Proliferation status using Ki-67 staining. Lower left panel: Demonstration of presence of H3K27M mutation in BXdmgl cells. Lower middle panel: Status of H3K27me trimethylation in BXdmgl cells. Lower right panel: Western blot on histone protein isolation demonstrating typical reduction of H3K27me after exposure to GSK126. (B, C) BXdmgl proliferation is inhibited by GSK126 with an IC50 of 10,36µM, a comparable sensitivity to the other DMG cells (see Figure 2). One way ANOVA (n=3, p<0.0001), Bonferroni's multiple comparisons post-test. (D) BXdmgl cells are not sensitive to exposure to cholesterol biosynthesis inhibitors. (E) Combo treatment of GSK126 and Atorvastatin (Ator) shows stronger growth inhibition than GSK126 or Atorvastatin alone. Combo effect is visible at low doses and is lost at higher doses of GSK126 because of its cytotoxicity alone at these concentrations. One way ANOVA (n=3, p<0.0001), Bonferroni's multiple comparisons post-test. *ns,* non-significant; *, p<0.05; **, p<0.01; ***, p<0.001.

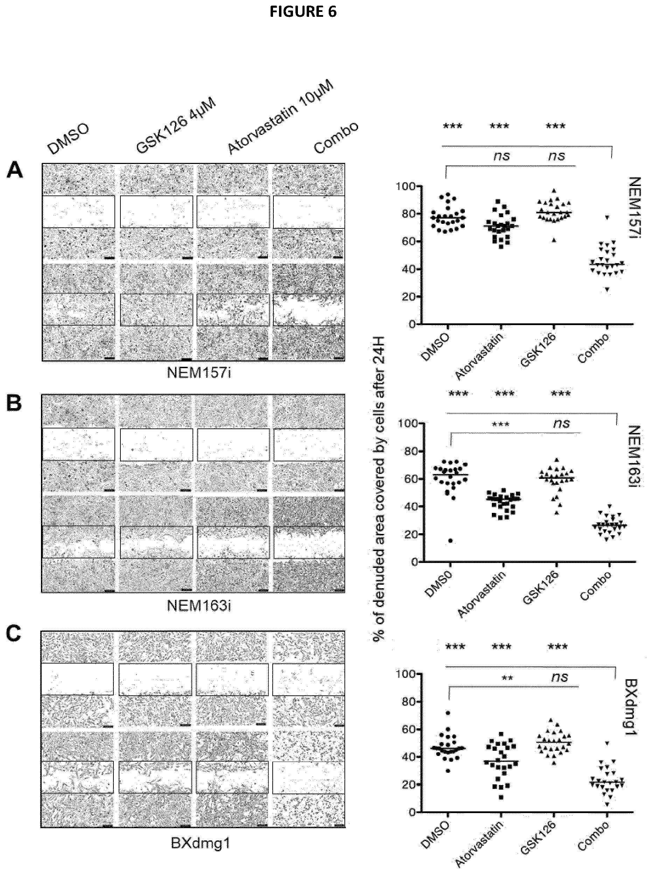

**Figures 6A****-C:** show the synergistic effect of GSK126 and inhibitors of cholesterol biosynthesis pathway enzymes on DMG cell migration. (A-C) Cell migration impairment in the combo treatment revealed by a wound scratch assay in the NEM157i (A) and NEM163i (B) cells and in the BXdmgl primary cells (C). One way ANOVA (n=3, p<0.0001), Bonferroni's multiple comparisons post-test. *ns,* non-significant; *, p<0.05; **, p<0.01; ***, p<0.001.

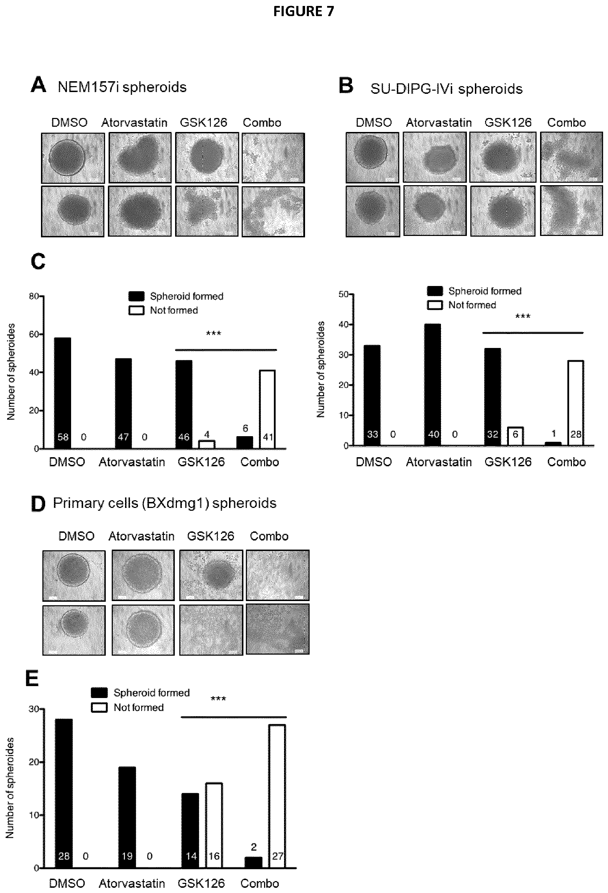

**Figures 7A****-E:** show the synergistic effect GSK126 and inhibitors of cholesterol biosynthesis pathway enzymes on DMG spheroid formation. (A, B, D) Phase contrast micrographs of representative spheroids derived from indicated DMG glioma cells after exposure to indicated inhibitors. (C, E) Statistical analysis of treatment effects on the formation of spheroids derived from the DMG cell line shown above the corresponding graph. Phenotypic criterions were spheroid formed or not formed (dispersed cells). Fisher' Exact test was used to compare relevant treatments and the combination (combo). GSK126 dose was 20µM for NEM157i and 15µM for the others. ***, p<0.001.

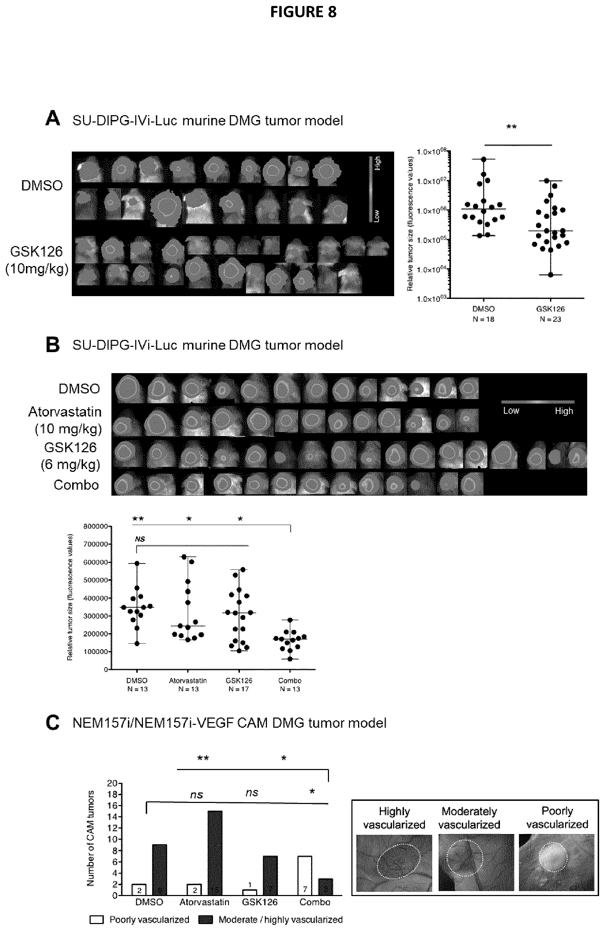

**Figures 8A****-C:** show the synergistic effect GSK126 and inhibitors of cholesterol biosynthesis pathway enzymes on DMG tumor development in mice. (A) Orthotopic tumor growth inhibition of SU-DIPG-IVi-Luc cells (expressing the Luciferase) implanted in the brainstem of NOD/LtSz-scid IL2R gamma (NSG) mice after GSK126 intraperitoneal treatments. Tumor growth inhibition is demonstrated for all mice by bioluminescence live imaging and statistical analysis after the indicated treatments (solvent control: DMSO; GSK126). Grey scale bar indicates level of expression, light grey values equal low and dark grey values high expression. Parametric t Student test (DMSO: n=18; GSK126: n=23) (B) The same approach is performed at a non-cytotoxic dose of GSK126 in combination with a non-cytotoxic dose of Atorvastatin. The combination of both drugs shows significant growth inhibitory effects, whereas single treatments are not effective. Bars indicate median plus range. One way ANOVA (DMSO: n=13; Atorvastatin: n=13; GSK126: n=17; Combo: n=13; p<0.0001), Bonferroni's multiple comparisons post-test.(C) Similar tumor growth inhibitory results were obtained using a chick CAM DMG model where a less angiogenic phenotype of implanted tumors was observed in the combo treatment, whereas single treatments had no anti-angiogenic effect Two-sided Fisher's exact test. *ns,* non-significant; *, p<0.05; ** p<0.01.

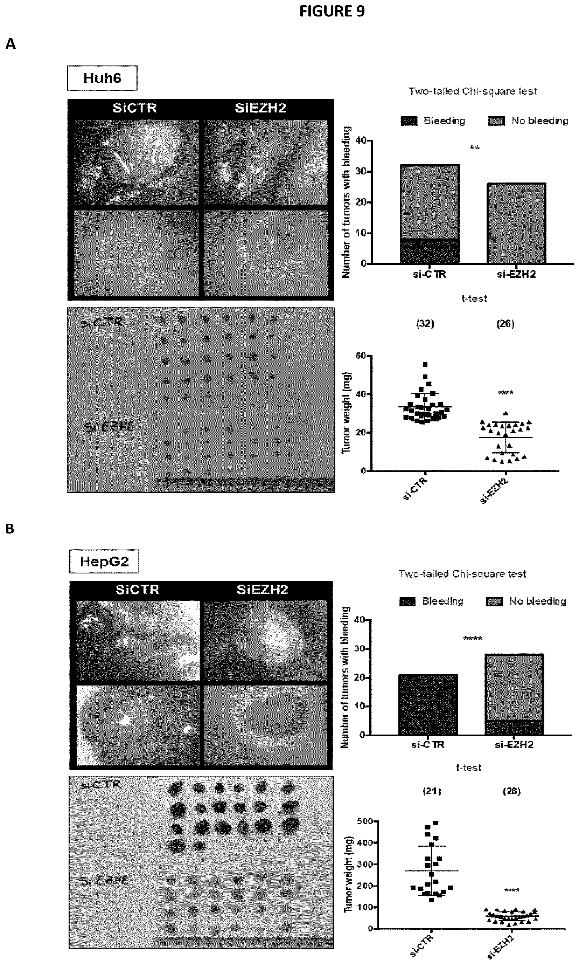

**Figures 9A****-B:** show that EZH2 silencing impedes hepatoblastoma development *in vivo.* (A-B) Huh6 cells (A) or HepG2 cells (B) were transfected with a control siRNA (SiCTR) or a siRNA against EZH2 (SiEZH2). 24 hours later, cells were collected and grafted on the chick chorioallantoic membrane (CAM) at day 10 of embryonic development. Tumor growth was monitored from day 11 to day 16. For both cell lines and the indicated condition, top left panels: representative pictures of tumors having grown on CAM (Lane 1) at day 17 and after resection and Paraformaldehyde fixation (lane 2). For both cell lines, top right panels: The number of CAMs presenting or not bleeding at Day 7 in SiCTR tumors *versus* SiEZH2 tumors is shown as bars. Two-sided Fisher's exact test. For both cell lines, bottom panels: On day 6 after SiEZH2 or SiCTR-transfected Huh6 cell implantation, tumors were resected and weighed. Left panels: representative pictures of extracted tumors in the indicated condition. Right panel: bars represent means +/- SD of tumor weight in mg. The total number of eggs analyzed per group is indicated in brackets above the corresponding condition. Parametric t Student test. **, p<0.01; ****, p<0.0001.

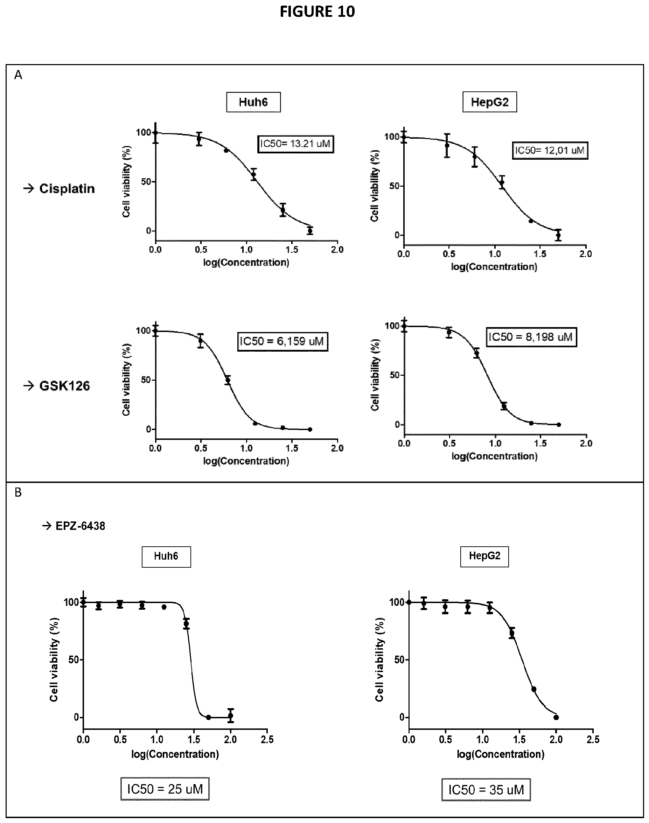

**Figures 10A****-B:** show the effect of Cisplatin alone or two EZH2 inhibitors on the survival of hepatoblastoma cells. (A-B) Huh6 and HepG2 cells were grown for 48 h in presence of increasing concentrations of Cisplatin (A), GSK126 (A) or EPZ-6438 (Tazemetostat, B) as indicated. Cell survival and IC50 were measured using the CellTiter 96^{®} AQᵤₑₒᵤₛ One Solution Cell Proliferation Assay from Promega. IC50 for the corresponding condition is as shown.

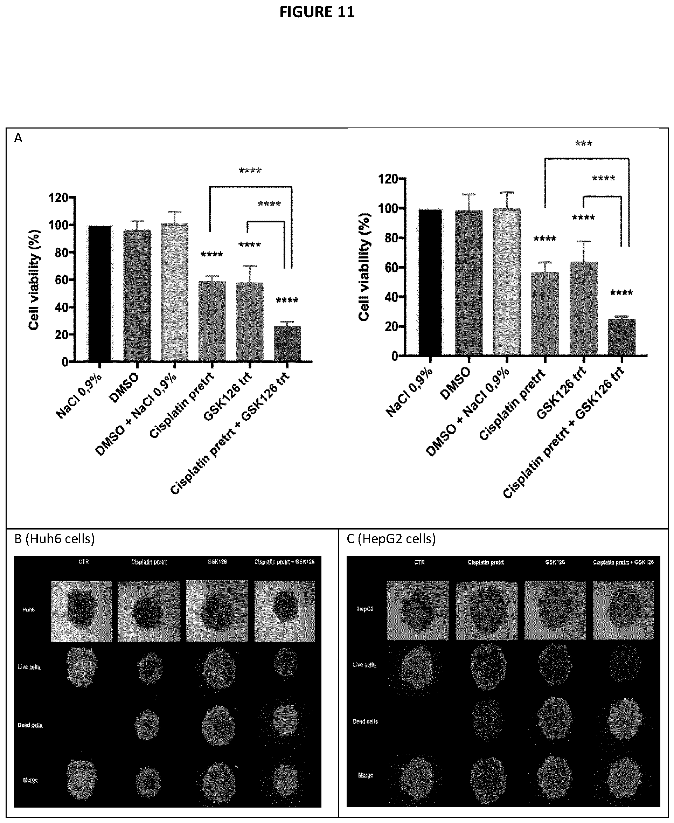

**Figures 11A****-C:** show the effect of combining Cisplatin and GSK126 on the viability of hepatoblastoma cells in 2D and 3D culture conditions. (A-B) Huh6 and HepG2 cells were grown for 72 h in classical 2D culture condition or as tumor spheroids in presence of GSK126 alone, Cisplatin alone, or a combination of both as indicated. The drug concentrations were selected to kill 50% of hepatoblastoma cells when used alone (see IC50 for each cell line and drug in Figure 10). (A) Cell survival was measured using the CellTiter 96^{®} AQᵤₑₒᵤₛ One Solution Cell Proliferation Assay from Promega (n=5, One-way ANOVA, p<0.0001), Sidak's multiple comparisons post-test. (B) Live cells were stained in green with Calcein-AM substrate (https://pubchem.ncbi.nlm.nih.gov/compound/Calcein-AM) and dead cells were stained in red using Ethidium homodimer (EthD-1, https://pubchem.ncbi.nlm.nih.gov/compound/12328897). Data show an additional antitumor effect of GSK126 and cisplatin *in vitro* on hepatoblastoma cells growing in 2D and 3D conditions. ***, p<0.001; ****, p<0.0001.

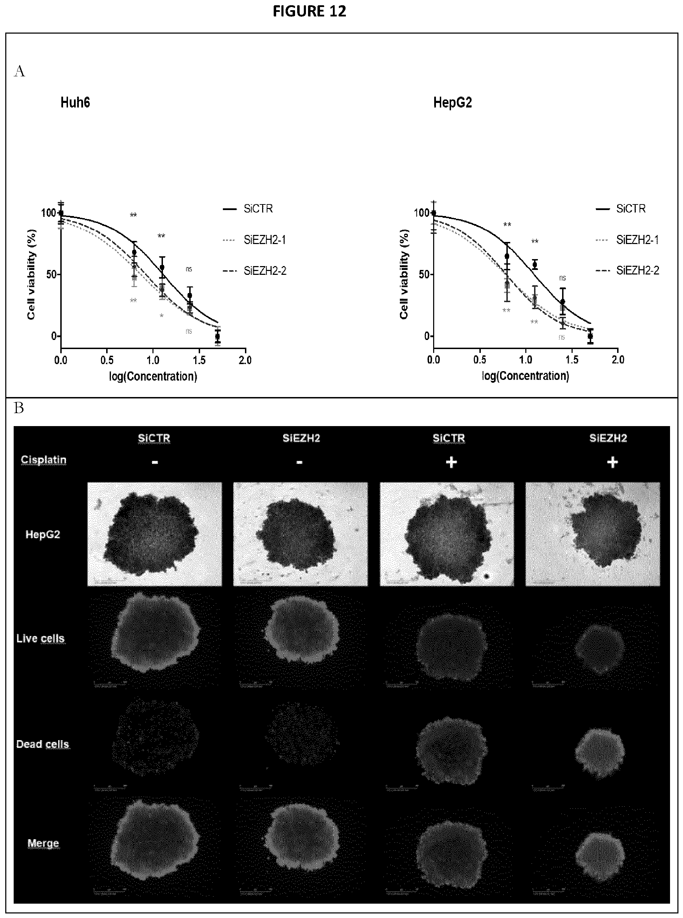

**Figures 12A****-B:** show that EZH2 depletion increases the sensitivity of hepatoblastoma cells to Cisplatin. (A) Huh6 (on the left) and HepG2 (on the right) cells were transfected with Control (SiCTR) or two different SiEZH2(SiEZH2-1 and SiEZH2-2) were grown for 72 h in presence of increasing concentrations of Cisplatin. Cell survival was measured using the CellTiter 96^{®} AQᵤₑₒᵤₛ One Solution Cell Proliferation Assay from Promega. (n=4, Two-way ANOVA, p<0.0001), Sidak's multiple comparisons post-test. Light grey asterisks: significant variations in cell viability between control cells and SiEZH2-1 transfected cells. Black asterisks: significant variations in cell viability between control cells and SiEZH2-2 transfected cells. (B) Control (SiCTR) or siEZH2-transfected HepG2 cells were grown for 48 h as tumor spheroids in presence of 12 µM of Cisplatin (=IC50). Live cells were stained in green with Calcein-AM substrate and dead cells were stained in red using EthD-1 (see Figure 11B). One representative illustration of 3 independent experiments. ns, non-significant; *, p<0.05; **, p<0.01.

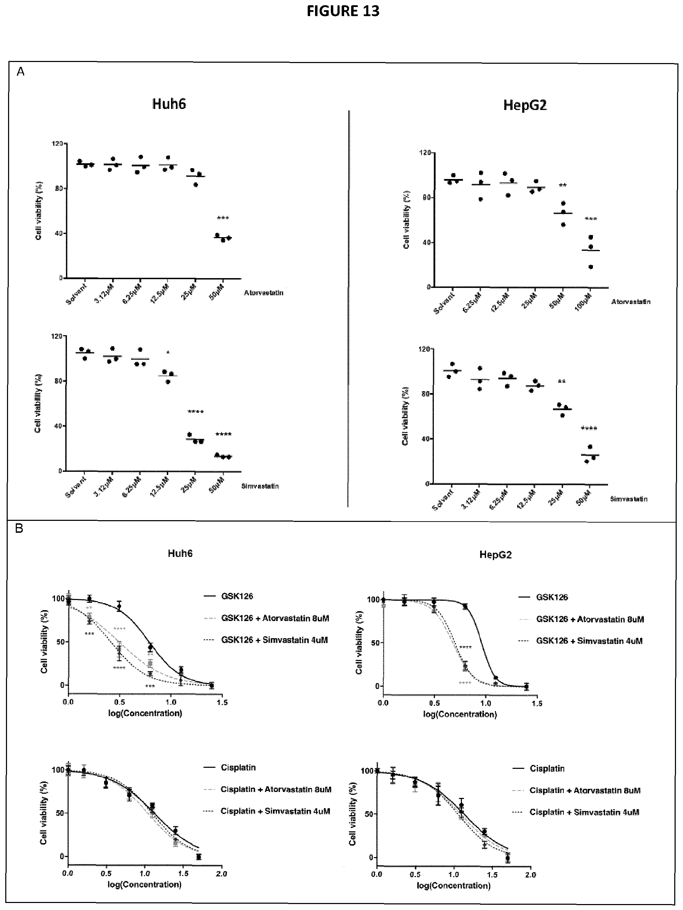

**Figures 13A****-B:** show that the statins synergize with GSK126 to eradicate hepatoblastoma cells. (A) Huh6 cells (on the left) and HepG2 cells (on the right) were treated for 72 h in presence of increasing concentrations of Simvastatin or of Atorvastatin (n=3, One-way ANOVA, p<0.0001; Sidak's multiple comparisons post-test, *, p <0.05; **, p< 0.01; ***, p <0.001; ****, p< 0.0001. (B) Huh6 cells (on the left) and HepG2 cells (on the right) were treated for 72 h in presence of increasing concentration of GSK126 (top panels) or of cisplatin (bottom panels) in absence of or in presence of 4 µM of Simvastatin or of 8 µM of Atorvastatin. (n=3, Two-way ANOVA, p<0.0001, Sidak's multiple comparisons post-test. (A-B) Cell viability and IC50 were measured in all conditions using the CellTiter 96^{®} AQᵤₑₒᵤₛ One Solution Cell Proliferation Assay (Promega). Light grey asterisks: significant variations in cell viability between GSK126 alone and GSK126 combined with the indicated concentrations of Atorvastatin. Black asterisks: significant variations in cell viability between GSK126 alone and GSK126 combined with the indicated concentrations of Simvastatin. * p<0.05, ** p<0.01, *** p<0.001, **** p<0.0001.

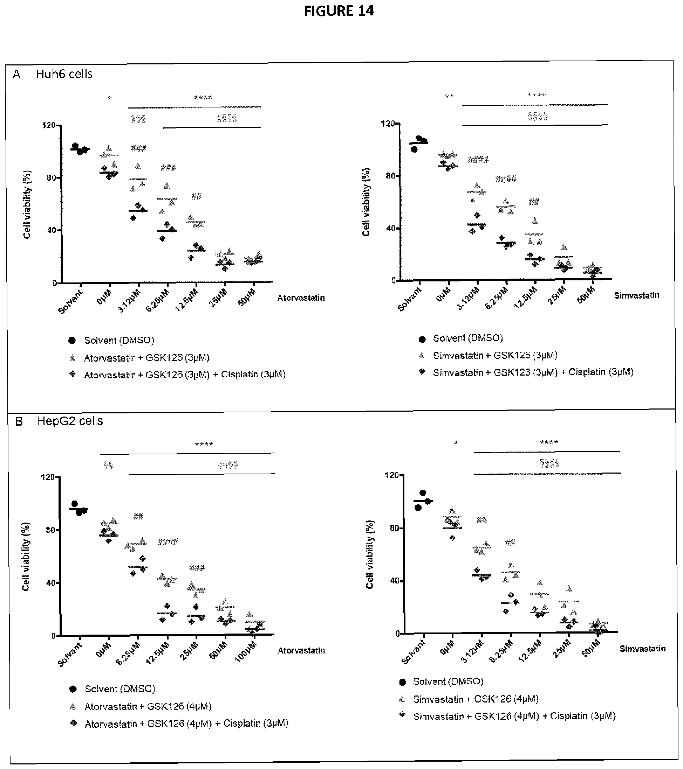

**Figures 14A****-B:** show that the statins synergize with GSK126 in absence of or in presence of Cisplatin to eradicate hepatoblastoma cells *in vitro.* (A-B) Huh6 cells (A) and HepG2 cells (B) were treated for 72 h by 3 µM of GSK126 in combination or not with 3µM of Cisplatin in presence of increasing concentrations of Simvastatin (right panels) or of Atorvastatin (left panels). Cell viability was measured in each condition using the CellTiter 96^{®} AQᵤₑₒᵤₛ One Solution Cell Proliferation Assay from Promega (n=3, Two-way ANOVA, p<0.0001; Sidak's multiple comparisons post-test. §: significant variations in cell viability between solvent control (DMSO) and GSK126 alone or GSK126 combined with any of the tested concentrations of the indicated statin. *: significant variations in cell viability between solvent control (DMSO) and GSK126+Cisplatin or GSK126+Cisplatin combined with any of the tested concentrations of the indicated statin. #: significant variations in cell viability between GSK126 and GSK126+Cisplatin at the indicated concentration of the indicated statin. *, p<0.05; **, p<0.01; ****, p<0.0001; ^{##}, p<0.01; ^{###}, p<0.001; ^{####}, p<0.0001; ^{§§}, p<0.01; ^{§§§}, p<0.001; ^{§§§§}, p<0.0001;

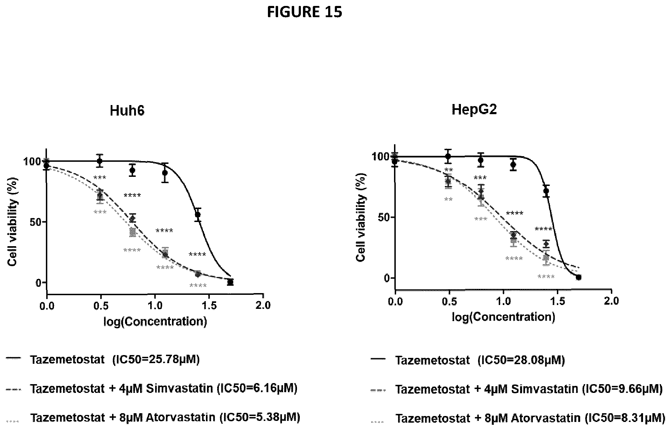

**Figures 15****:** shows that statins strongly increase the sensitivity of hepatoblastoma cell to Tazemetostat (EPZ-6438). Huh6 cells (on the left) and HepG2 cells (on the right) were treated for 72 h in presence of increasing concentrations of Tazemetostat in absence of or in presence of 8 µM of Atorvastatin or of 4µM of Simvastatin (as indicated in the legend below each graph). Cell viability and IC50 (see value in brackets for each condition) were measured for each condition using the CellTiter 96^{®} AQᵤₑₒᵤₛ One Solution Cell Proliferation Assay from Promega (n=3, Two-way ANOVA, p<0.0001; Sidak's multiple comparisons post-test,. Black asterisks: significant variations in cell viability between Tazemetostat alone and Tazemetostat in presence of Simvastatin. Light grey asterisks: significant variations in cell viability between Tazemetostat alone and Tazemetostat in presence of Atorvastatin. *, p<0.05; **, p<0.01; ***, p<0.001; ****, p<0.0001.

### DETAILED DESCRIPTION

The present invention thus relates to methods of treating and/or preventing cancers as well as synergistic compositions and combinations for use in a method of treating and/or preventing cancers and tumors associated with histone methyltransferase EZH2 comprising administering to a subject in need a therapeutically effective amount of the composition, wherein said composition comprises the combination of EZH2 inhibitors and one statins. Said synergistic compositions or combinations are referred herein after as bi-therapies or combinatory therapies.

EZH2 inhibitors are typically S-Adenosyl-L-methionine (SAM) competitive inhibitors of EZH2, and carry 2-pyridone moiety or tetramethylpiperidinobenzamide moiety as described *inter alia* in Fioravanti R. et al. (Chem. Rec. 2018, 18, 1818-1832).

By way of example and without any limitations, EZH2 inhibitors may include catalytic 2-pyridone EZH2 inhibitors with a bicyclic heteroaromatic ring as the central scaffold, such as GSK126 or GSK2816126 (1-[(2S)-butan-2-yl]-N-[(4,6-dimethyl-2-oxo-1H-pyridin-3-yl)methyl]-3-methyl-6-[6-(1-piperazinyl)-3-pyridinyl]-4-indolecarboxamide), UNC1999 (N-[(6-methyl-2-oxo-4-propyl-1H-pyridin-3-yl) methyl]-1-propan-2-yl-6-[6-(4-propan-2-ylpiperazin-1-yl)pyridin-3-yl]indazole-4-carboxamide), EPZ005687 (1-cyclopentyl-*N*-[(4,6-dimethyl-2-oxo-1*H*-pyridin-3-yl)methyl]-6-[4-(morpholin-4-ylmethyl)phenyl]indazole-4-carboxamide), and Ell (6-cyano-N-[(4,6-dimethyl-2-oxo- 1H-pyridin-3-yl)methyl]-1-pentan-3-ylindole-4-carboxamide). We can also cite MC3629, a simplified analogue of EPZ005687 and GSK126, comprising pyrazole- and pyrrole-based compounds linked through an amide bond to the 2-pyridone moiety, GSK926 (*N*-[(4,6-dimethyl-2-oxo-1*H*-pyridin-3-yl)methyl]-6-[6-(4-methylpiperazin-1-yl)pyridin-3-yl]-1-propan-2-ylindazole-4-carboxamide), GSK343 (*N*-[(6-methyl-2-oxo-4-propyl-1H-pyridin-3-yl)methyl]-6-[2-(4-methylpiperazin-1-yl)pyridin-4-yl]-1-propan-2-ylindazole-4-carboxamide), and GSK503 (*N*-[(4,6-dimethyl-2-oxo-1*H*-pyridin-3-yl)methyl]-3-methyl-6-[6-(4-methylpiperazin-1-yl)pyridin-3-yl]-1-propan-2-yl indole-4-carboxamide). We can further cite CPI-360 carrying a 2-pyridone moiety linked to the indole nucleus through an amide function, its analog CPI-169 and CPI-1205 (N-[(4-methoxy-6-methyl-2-oxo-1H-pyridin-3-yl)methyl]-2-methyl-1-[(1R)-1-[1-(2,2,2-trifluoroethyl) piperidin-4-yl]ethyl] indole-3-carbo xamide).

We may also cite Tazemetostat also named EPZ-6438 or E7438 (*N*-[(4,6-dimethyl-2-oxo-1*H*-pyridin-3-yl)methyl]-3-[ethyl(oxan-4-yl)amino]-2-methyl-5-[4-(morpholin-4-ylmethyl)phenyl] benzamide, as well as two orally available benzamidomethyl-2-pyridone analogues of tazemetostat: EPZ011989 which is also designated 1598383-40-4 (*N*-[(4,6-dimethyl-2-oxo-1*H*-pyridin-3-yl)methyl]-3-[ethyl-[4-[2-methoxyethyl(methyl)amino] cyclohexyl]amino]-2-methyl-5-(3-morpholin-4-ylprop-1-ynyl) benzamide) and ZLD1039 also designated 1826865-46-6 (3-[ethyl(oxan-4-yl)amino]-2-methyl-*N*-[(1-methyl-3-oxo-5,6,7,8-tetrahydro-2*H*-isoquinolin-4-yl)methyl]-5-[6-(4-methyl piperazin-1-yl)pyridin-3-yl]benzamide). Other analogs of tazemetostat include EBI-2511 (*N*-[(4,6-dimethyl-2-oxo-1*H-*pyridin-3-yl)methyl]-5-ethyl-6-[ethyl(oxan-4-yl)amino]-2-(1-propan-2-ylpiperidin-4-yl)-1-benzofuran-4-carboxamide), pinometostat ((2*R*,3*R*,4*S*,5*R*)-2-(6-aminopurin-9-yl)-5-[[[3-[2-(6-*tert*-butyl-1*H*-benzimidazol-2-yl)ethyl]cyclobutyl]-propan-2-ylamino]methyl]oxolane-3,4-diol), lirametostat (*N*-[(4-methoxy-6-methyl-2-oxo-1*H*-pyridin-3-yl)methylethyl-1-[(1*R*)-1-[1-(2,2,2-trifluoroethyl)piperidin-4-yl]ethyl]indole-3-carboxamide).

We may further cite JQEZ5 also named JQE5 (1-isopropyl-N-((6-methyl-2-oxo-4-propyl-1,2-dihydropyridin-3-yl)methyl)-6-(6-(4-methylpiperazin-1-yl) pyridin-3-yl)-1H-pyrazolo[3,4-b]pyridine-4-carboxamide), PROTAC (PROteolysis TArgeting Chimeric) MS1943 (See, Feral et al., Adv. Therap. 2020, 3, 2000148), DZNep ((1S,2R,5R)-5-(4-aminoimidazo[4,5-c]pyridin-1-yl)-3-(hydroxymethyl) cyclopent-3-ene-1,2-diol)), MC1947, and MC1948.

Furthermore, we may cite PF-06821497 compound which is a catalytic EZH2 inhibitor comprising a 3,4-dihydroisoquinoline moiety in addition to the 2-pyridone moiety. The full chemical name is 5,8-dichloro-2-[(4-methoxy-6-methyl-2-oxo-1*H*-pyridin-3-yl)methyl]-7-[(*R*)-methoxy(oxetan-3-yl)methyl]-3,4-dihydroisoquinolin-1-one.

Other selective EZH2 inhibitors which may be used in synergistic compositions according to the present invention include:
- 5.8-dichloro-2-[(4-methoxy-6-methyl-2-oxo-1,2-dihydro-pyridin-3-yl)methyl]-7-[methoxy(oxetan-3-yl)methyl]-3,4-dihydroisoquinolin-1(2/-/)-one;
- 5.8-dichloro-2-[(4-methoxy-6-methyl-2-oxo-1,2-dihydro-pyridin-3-yl)methyl]-7-[(R)-methoxy(oxetan-3-yl)methyl]-3,4-dihydroisoquinolin-1(2H)-one;
- 5.8-dichloro-2-[(4-methoxy-6-methyl-2-oxo-1,2-dihydro-pyridin-3-yl)methyl]-7-[(S)-methoxy(oxetan-3-yl)methyl]-3,4-dihydroisoquinolin-1(2/-/)-one;
- 5-bromo-8-chloro-2-[(4,6-dimethyl-2-oxo-1,2-dihydropyridin-3-yl)methyl]-7-(1 ,4-dimethyl-f/-/-1,2,3-triazol-5-yl)-3,4-dihydroisoquinolin-1 (2/-/)-one; 5, 8-dichloro-7-(3,5-dimethyl-1,2-oxazol-4-yl)-2-[(4,6-dimethyl-2-oxo-1,2-dihydropyridin-3-yl)methyl]-3,4-dihydroisoquinolin-1{2H)-one;
- N-[(4,6-dimethyl-2-oxo-1,2-dihydropyridin-3-yl)methyl]-5-[ethyl(tetrahydro-2H-pyran-4-yl)amino]-4-methyl-4'-(morpholin-4-ylmethyl)biphenyl-3-carboxamide;
- N-[(4,6-dimethyl-2-oxo-1,2-dihydropyridin-3-yl)methyl]-5-[ethyl(tetrahydro-2H-pyran-4-yl)amino]-4-methyl-4'-(morpholin-4-ylmethyl)biphenyl-3-carboxamide;
- N-[(4-methoxy-6-methyl-2-oxo-1, 2-dihydropyridin-3-yl)methyl]-2-methyl-1-[(1R)-1-[1-(2,2,2-trifluoroethyl)piperidin-4-yl]ethyl]-1H-indole-3-carboxamide;
- N-(4,6-dimethyl-2-oxo-1, 2-dihydropyridin-3-ylmethyl)-1-isopropyl-3-methyl-6-[6-(4-methylpiperazin-1-yl)pyridin-3 -yl]-1H-indole-4-carboxamide;
- N-[(6-methyl-2-oxo-4-propyl-1,2-dihydropyridin-3-yl)methyl]-6-[2-(4-methy lpiperazin-1-yl)pyridin-4-yl]-1-(propan-2-yl)-1H-indazole-4-carboxamide.

Said one statin may be elected among the group consisting of atorvastatin, fluvastatin, pitavastatin, pravastatin, mevastatin, rosuvastatin, simvastatin, cerivastatin, and/or analogues thereof. Statins are available upon prescription. There are several more under clinical investigation. Said one statin used in bi-therapies according to the present invention however preferably does not comprise lovastatin and thus is devoid of any lovastatin.

Statins are a group of compounds that are 3-hydroxy-3-methyl-glutaryl-coenzyme A reductase inhibitors (also known as HMG-CoA inhibitors or HMGCR inhibitors) that function as lipid-lowering compounds. HMG-CoA is an enzyme {i.e., NADH-dependent (EC 1.1.1.88), NADPH-dependent (EC 1.1.1.34)) that is the rate-limiting enzyme of the mevalonate pathway, *i.e.,* the metabolic pathway that produces cholesterol and other isoprenoids. HMG-CoA is normally suppressed by cholesterol that is derived from the internalization and degradation of low-density lipoprotein (LDL) via the LDL receptor, as well as oxidized species of cholesterol. Competitive inhibition of HMG-CoA by statins initially reduces cholesterol production, and, as an adaptive response, the reduced cellular cholesterol level triggers the sterol regulatory element-binding protein (SREBP)-mediated activation of gene expression, including that of LDL receptors (LDLRs) in the liver and other tissues, which leads to an increased uptake of LDL from the circulation, hence lowering blood cholesterol levels.

Synergistic compositions or bi-therapies according to the present invention are effective for treating and/or preventing cancers and tumors associated with histone methyltransferase EZH2. They have been showed to produce beneficial changes in a patient's cancer status. The changes can be either subjective or objective and can relate to features such as symptoms or signs of the cancer being treated, such as reducing the number of cancer cells, the growth of the cancer cells, the size of cancer tumors, the resistance of the cancer cells to another cancer drug, and/or preventing the deterioration of the patient's status.

As used herein, patient or subject may be used interchangeably and refers to a human in need of cancer treatment or at risk of developing a cancer/tumor.

Cancers and tumors associated with histone methyltransferase EZH2 may be selected in particular among hepatoblastoma, Diffuse Intrinsic Pontine Glioma (DIPG), Diffuse Midline Glioma (DMG), bladder cancer, bone cancer, brain cancers, breast cancer, malignant lymphoma, rhabdoid tumor, leukemia, lung cancer, stomach cancer, prostate cancer, colorectal cancer, esophageal cancer, ovarian cancer, uterine cancer, liver cancer, testicular cancer, pancreatic cancer, renal cancer, rectal cancer, thyroid cancer, skin cancer, head & neck cancer. Said tumors and cancers are preferably not associated with endothelial to mesenchymal transition associated pathology.

Synergistic bi-therapy compositions or combinatory therapies according to the present invention are preferably administered to relapsed or refractory patients after anticancer standard treatment, radiotherapy or other first or second line of cancer therapies, as some of these cancers or tumors may be resistant to certain anti-cancer drug.

Indeed, as demonstrated by the Applicants in the Examples below, said bi-therapies have a significant synergistic therapeutic effect: the therapeutic and/or the beneficial effects that are produced when GSK126 and one statin are administered in combination or co-administered are greater compared to when they are administered alone and greater to the effects produced by a sum of the effects of the individual compounds (*i.e.,* an effect that is greater than an additive effect). In particular, an unexpected efficacy of the combinatory therapy or bi-therapy has been evidenced notably on cell migration and adhesion. Methods of identifying synergistic effects are discussed in various publications such as Foucquier J. et al. (Pharmacology Research & Perspectives (2015) (3)3:e00149).

Because of the synergistic effects of the bi-therapy compositions, therapeutically or prophylactically effective amounts of EZH2 inhibitor which have been administered or co-administered in combination with one statin to a patient may be much lower than the doses administered so far in current clinical phases. Effective amounts of GSK126 may be comprised between 1 and 10mg/kg of the patient. Such reduction of the dosage regimen of EZH2 inhibitor within the bi-therapy of the present invention has a significant impact in greatly reducing the general toxicity of the drug to patient, while maintaining efficient anti-cancerous effects including the delay or inhibition of the cancer cell, the inhibition of the tumor growth, progression and/or metastasis, the reduction of tumor size, the induction of cell death, the increase of the mean time of survival, and the sensitization of cancer cell to an anticancer drug to which it has become or is resistant.

Synergistic compositions and methods of treating according to the present invention may further comprise an effective amount of one or more anticancer drugs or chemotherapeutic agents.

These are referred herein below as tri-therapies. Said one or more anticancer drugs may be chosen among platinum compounds or platinum-based agents, tyrosine kinase inhibitors, taxane derivatives, topoisomerase inhibitors, hormone therapeutic agents, anti-androgen drugs, androgen receptor agents, anti-angiogenesis agents, immunotherapeutic preparations, anti-inflammatory drugs, radiotherapeutic agents, biological preparations having anticancer effects, anticancer preparations made from natural substances.

Preferred anticancer drug used in the tri-therapies of the present invention are platinum compounds or platinum-based agents, such as for example cisplatin, carboplatin, and oxaliplatin, satraplatin, picoplatin, nedaplatin, triplatin, and/or lipoplatin. Applicants demonstrated in the Examples below that administering tri-therapy combinations comprising an effective amount of GSK126, statins with a further anticancer drug, such as said platinum compounds, provide superior therapeutic effects, thereby resulting in a substantial inhibition cancer cell growth, inhibition cancer cell metastasis, decrease tumor size, increased survival time of the subject, and more generally in a substantial improvement of one or more signs and/or symptoms of cancer. It is possible that such tri-therapy combinations reverse or reduces cancer cell resistance to the anticancer drug, and/or sensitize cancer cells to the anticancer drug, but the mechanism of action is unknown.

In particular the Applicants clearly demonstrated in the Example 2 that the methyl-transferase EZH2 is a key oncogene in hepatoblastoma and a relevant therapeutic target for the treatment of this pediatric liver tumor. Using genetic or drug-based pharmacological approaches to inactivate EZH2 functions, it was showed that EZH2 protein is required for the 2D and 3D growth and survival of hepatoblastoma cells in vitro and the development of aggressive and angiogenic tumor in the chick embryo model. Applicants found that the depletion of EZH2 increased the sensitivity of hepatoblastoma cells to cisplatin and that the combination of cisplatin with GSK126 had an additive effect on the elimination of tumoral cells in vitro. Used alone, GSK126 was also very efficient to kill hepatoblastoma cells in vitro at concentrations ranging from 5 to 10µM. These data thus clearly showed that such bi-therapies comprising for example cisplatin and GSK126 is efficient in first line of treatment could improve the chemotherapy efficiency, and in addition could also be useful as a second line of treatment for patients in relapse and presenting an acquired resistance to cisplatin.

In addition, Applicants implemented one tri-therapy according to the present invention comprising an EZH2 inhibitor, a statin (such as for example Simvastatin or Atorvastatin) and cisplatin and showed that the antitumor potential of GSK12 alone was also increased by the addition of Simvastatin or Atorvastatin, thereby demonstrating the therapeutic benefit of the tri-therapies.

Chemotherapeutic agents are well known in the art and include but are not limited to anthracenediones (anthraquinones) such as anthracyclines (*e.g*, daunorubicin (daunomycin; rubidomycin, doxorubicin, epirubicin, idarubicin, and valrubicin); tamoxifen and metabolites thereof such as 4-hydroxytamoxifen (afimoxifene) and N-desmethyl-4-hydroxytamoxifen (endoxifen); taxanes such as paclitaxel (taxol), docetaxel, cabazitaxel, hongdoushan A, hongdoushan B, hongdoushan C, baccatin I and baccatin II; alkylating agents (e.g., nitrogen mustards such as mechlorethamine (HN2), cyclophosphamide, ifosfamide, melphalan (L-sarcolysin), and chlorambucil); ethylenimines and methylmelamines (*e.g*, hexamethylmelamine, thiotepa, alkyl sulphonates such as busulfan, nitrosoureas such as carmustine (BCNU), lomustine (CCNLJ), semustine (methyl-CCN-U), and streptozoein (streptozotocin), and triazenes such as decarbazine (DTIC; dimethyltriazeno-imidazolecarboxamide); antimetabolites (*e.g*, folic acid analogs such as methotrexate (amethopterin), pyrimidine analogs such as fluorouracil (5-fluorouracil; 5-FU), floxuridine (fluorodeoxyuridine; FUdR), and cytarabine (cytosine arabinoside), and purine analogs and related inhibitors such as mercaptopurine (6-mercaptopurine; 6-MP), thioguanine (6-thioguanine; 6-TG), and pentostatin or 2'-deoxycofonnycin); natural products, *e.g*, vinca alkaloids, such as vinblastine (VLB) and vincristine, epipodophyllotoxins such as etoposide and teniposide; antibiotics such as dactinomycin (actinomycin D), bleomycin, plicamycin (mithramycin), and mitomycin (mitomycin Q); enzymes such as L-asparaginase; substituted ureas such as hydroxyurea; methyl hydrazine derivatives such as procarbazine (N-methylhydrazine; MIH); and/or adrenocortical suppressants such as mitotane and aminoglutethimide.

Anti-androgen drugs are drugs which alter the androgen pathway by blocking the androgen receptors, competing for binding sites on the cell's surface, or affecting or mediating androgen production. Anti-androgen drugs include, but are not limited to, enzalutamide, abiraterone, bicalutamide, flutamide, nilutamide, apalutamide, finasteride, dutasteride, alfatradiol, and combinations thereof.

Radiotherapeutic agents are well known in the art and comprise external-beam radiation therapy and/or internal radiation therapy. External beam radiation therapy delivers radioactive beams of high energy X-rays and/or gamma rays to a patient's tumor, whereas internal radiation therapy delivers radioactive atoms to a patient's tumor. Both external beam radiation therapy and internal radiation therapy are used to suppress tumor growth or kill cancer cells by delivering a sufficient quantity of radioactivity to the target site. In some embodiments, the radiotherapeutic agent comprises a radioactive atom and is complexed with a biologic or synthetic agent to increase delivery to the target site. Radiotherapeutic agents may thus be coupled to targeting moieties, such as antibodies, to improve the localization of radiotherapeutic agents to cancerous cells.

Said tri-therapy compositions preferably do not comprise any inhibitors of RORy (Retinoic acid receptor-related orphan receptor y), do not comprise any anti-VEGF agent such as sunitinib, or agents such as gefitinib, erlotinib, sorafenib, sunitinib, dasatinib, lapatinib, nilotinib, bortezomib, or salinomycin, do not comprise VEFG/VEGFR inhibitors, do not comprise BCL2 inhibitors or LSD1 inhibitors.

The present invention thus also provides a composition for use in a method of treating and/or preventing tumors associated with methyltransferase EZH2, comprising administering to a subj ect in need a therapeutically effective the bi-therapy or tri-therapy as described above, wherein said bi-therapy or tri-therapy is administered to relapsed or refractory patients after anticancer standard treatment, radiotherapy or other first or second line of cancer therapies.

The present invention further provides pharmaceutical compositions comprising the bi-therapy or tri-therapy combinations as described above and a pharmaceutically acceptable excipient and/or carrier and/or diluent. The pharmaceutical compositions may also optionally comprise one or more anticancer drugs. Any pharmaceutically acceptable carrier and/or excipient and/or diluent suitable for the formulation of the present composition and the desired administration is contemplated herein.

Typically, such pharmaceutically acceptable excipient may include a salt or a diluent, and may further comprise pharmaceutical cryoprotectant, such as glucose, sucrose, trehalose, lactose, sodium glutamate, PVP, HPpCD, CD, glycerol, maltose, mannitol, and saccharose.

The pharmaceutical compositions of the present invention include formulations suitable for topical, parenteral, pulmonary, nasal, rectal, or oral administration. The most suitable route of administration in any given case will depend in part on the nature and severity of the cancer condition and also optionally the stage of the cancer. Preferred pharmaceutical compositions are formulated for parenteral administration, and most preferably for intravenous administration. Both immediate release and sustained release dosage forms are within the scope of the present invention.

Pharmaceutical compositions of the present invention may be prepared by any of the methods well-known in the art of pharmacy. Pharmaceutically acceptable carriers suitable for use with the present invention include any of the standard pharmaceutical carriers, buffers and excipients, including phosphate-buffered saline solution, water, and emulsions (such as an oil/water or water/oil emulsion), and various types of wetting agents and/or adjuvants. Suitable pharmaceutical carriers and their formulations are described in Remington's Pharmaceutical Sciences (Mack Publishing Co., Easton, 19th ed. 1995). Preferred pharmaceutical carriers depend upon the intended mode of administration of the active agent(s).

Pharmaceutical compositions are thus preferably formulated for parenteral administration by injection, for example by bolus injection or continuous infusion. Formulations for injection can be presented in unit dosage form, for example, in ampoules or in multi-dose containers, with an added preservative. Injectable compositions are preferably aqueous isotonic solutions or suspensions. Formulations may be sterilized and/or contain adjuvants, such as preserving, stabilizing, wetting or emulsifying agents, solution promoters, salts for regulating the osmotic pressure and/or buffers.

According to the present invention, the EZH2 inhibitor is advantageously co-administered with a statin and optionally anticancer agent to produce a synergistic effect on treatment and/or prevention of cancers and tumors. The EZH2 inhibitor, statins and as well as anticancer agent may be administered concomitantly (co-administered) or sequentially.

In case of tri-therapies, the EZH2 inhibitor and the statin may be administered to the patient in combination with an anticancer drug via the same or a different administration route, orally or parenterally (*e.g*, intravenously). For example, the EZH2 inhibitor and the satins are administered parenterally (*e.g*, intravenously), while the anticancer drug may be administered orally, or vice versa.

The patient may thus receive a therapeutically effective amount of GSK126 compound and one statin. Such administration may include providing effective amounts for a specified period of time, *e.g*, for about 1 to 24 hours or 1, 2, 3, 4, 5, 6, 7, 8, 9, 10, 12, 13, 14, 15, 16, 17, 18, 19, 20, 21, 22, 23, 24, 25, 26, 27, 28, 29, 30, 31 or more days, or in a specified sequence, e.g., administration of GSK126 and a statin followed by the administration of one or more anticancer drugs, or vice versa. The protocol of treatment may include sequential or simultaneous administration of two or more structurally different compounds. For example, two or more structurally different pharmaceutically active compounds can be co-administered by administering a pharmaceutical composition adapted for oral administration that contains two or more structurally different active pharmaceutically active compounds. As another example, two or more structurally different compounds can be co-administered by administering one compound and then administering the other compound. In some instances, the co-administered compounds are administered by the same route. In other instances, the co-administered compounds are administered via different routes. For example, one compound can be administered orally, and the other compound can be administered, *e.g*, sequentially or simultaneously, via intravenous or intraperitoneal injection.

Therefore, the pharmaceutical compositions may be prepared as a single medicament or separate medicaments comprising individual dosage units made by admixing the EZH2 inhibitor and one statins (*e.g*, atorvastatin, simvastatin, fluvastatin, rosuvastatin, mevastatin, cerivastatin, pravastatin, and/or pitavastatin), a pharmaceutically acceptable carrier and/or excipient or diluent, and optionally one or more anticancer drugs.

Pharmaceutical compositions or medicaments can be administered to a subject at a therapeutically effective dose to prevent, treat, sensitize, or control a cancer responsive to EZH2 inhibition. The pharmaceutical composition or medicament is administered to a subject in an amount sufficient to elicit an effective therapeutic response in the subject. An effective therapeutic response includes a response that at least partially arrests or slows the symptoms or complications of the cancer. An amount adequate to accomplish this is defined as a "therapeutically effective dose."

The dosage of active agents administered is dependent on the subject's body weight, age, individual condition, surface area or volume of the area to be treated and on the form of administration. The size of the dose also will be determined by the existence, nature, and extent of any adverse effects that accompany the administration of a particular formulation in a particular subject. Typically, a dosage of the active compound(s) of the present invention is a dosage that is sufficient to achieve the desired effect. Optimal dosing schedules can be calculated from measurements of active agent accumulation in the body of a subject. In general, dosage may be given once or more of daily, weekly, or monthly. Persons of ordinary skill in the art can easily determine optimum dosages, dosing methodologies and repetition rates. Optimum dosages, toxicity, and therapeutic efficacy of the compositions of the present invention may vary depending on the relative potency of the administered composition and can be determined by standard pharmaceutical procedures in cell cultures or experimental animals, for example, by determining the LD50 (the dose lethal to 50% of the population) and the ED50 (the dose therapeutically effective in 50% of the population). The dose ratio between toxic and therapeutic effects is the therapeutic index and can be expressed as the ratio, LD50/ED50. Agents that exhibit large therapeutic indices are preferred. While agents that exhibit toxic side effects can be used, care should be taken to design a delivery system that targets such agents to the site of affected tissue to minimize potential damage to normal cells and, thereby, reduce side effects.

Generally, an efficacious or effective amount of an composition is determined by first administering a low dose or small amount of the composition, and then incrementally increasing the administered dose or dosages, adding a second or third medication as needed, until a desired effect of is observed in the treated subject with minimal or no toxic side effects.

Single or multiple administrations of the compositions are administered depending on the dosage and frequency as required and tolerated by the patient. In any event, the composition should provide an efficient amount of the compositions of this invention to effectively treat the patient. Generally, the dose is sufficient to treat or ameliorate symptoms or signs of disease without producing unacceptable toxicity to the patient.

Throughout this application, various references are referred to and disclosures of these publications in their entireties are hereby incorporated by reference into this application to more fully describe the state of the art to which this invention pertains.

### EXAMPLES

### Example 1: Bi-therapies Efficiency for treating and/or preventing Diffuse Midline Glioma (DMG)

### Example 1.1: Methods

### Microarray analysis

The GSE50021 expression profile (35 DMG samples, 10 normal brain samples), was extracted from the Gene Expression Omnibus database. Raw reads were quantile normalized and log2 transformed. Expression values identified by ILMN_1652913 probe for EZH2 were extracted and analyzed using GraphPad Prism.

### DMG cell lines and primary BXdmg1 cells

DMG cell lines used in this study were NEM157i, NEM157i-VEGF, NEM163i, SU-DIPG-IVi, SU-DIPG-IVi-Luc and primary cells BXdmgl. Origin and lentiviral modification procedures (including immortalization) as well as culture conditions have been described herein below.

### Chemical inhibitors

EZH2 inhibitor GSK126, Atorvastatin, ACSS2 inhibitor and Terbinafine have been used (Selleckchemicals, Houston, USA).

### Western blots

Blots were performed using standard techniques (see below).

### Histone Extraction

Histone extraction was performed according to manufacturer's instructions (Histone Extraction Kit - ab113476, Abcam, Paris, France).

### Proliferation assays

Cell growth was measured with the In vitro Toxicology Assay kit (Sulforhodamine B, Sigma Aldrich, Saint Quentin Fallavier, France) according to manufacturer's instructions. Cells were plated at a density of 2000 cells per well in 96-well plates in triplicates. Absorbance was measured at 565 nm using the CLARIOstar multiplate reader (BMG Labtech, Champigny-sur-Marne, France) at indicated time points.

### Apoptosis assays

After exposure to drugs at indicated concentrations and times, cells were labeled with Annexin V-PE (BD Biosciences, Le Pont de Claix, France) and 7-AminoActinomycin D (7-AAD) (BD Bioscience) and analyzed using Flow Cytometry as described [4].

### Migration assays

2x104 cells/well were placed in a 96-well and incubated for 24h. An IncuCyte Wound Maker (96-pin wound making tool) was used to make scratches.

### Spheroid cultures

Standard culture methods for spheroid cultures were used for both DMG cell lines and hepatoblastoma cell lines.

### DMG in vivo models

Animal procedures were carried out in agreement with the European (directive 2010/63/UE) and French (decree 2013-118) guidelines. Mouse experiments have been authorized by local ethic commission and validated by the French Minister of Higher Education, Research and Innovation (APAFIS #13466-2019032112211281, authorization number B33063916).

The DMG NEM157i/NEM157i-VEGF CAM model has been described before Capdevielle V et al. Neuro Oncol 2019; 10.1093/neuonc/noz215).

A murine orthotopic model (SU-DIPG-IVi-Luc in NOD/LtSz-scid IL2R gamma mice) was developed based on the work of Mohammed et al. (Nat Med 2017;23(4):483-492).

### Chemical inhibitors

GSK126 is a highly selective EZH2 methyltransferase inhibitor with an IC50 of 9.9 nM, (>1000-fold selective for EZH2 over 20 other human methyltransferases). GSK126 (SelleckChem) was dissolved with DMSO (Dimethyl sulfoxide) and stored at -20°C. Three inhibitors of the cholesterol synthesis pathway have been used: Atorvastatin, an inhibitor of HMG-CoA reductase, ACSS2 inhibitor which targets acetate-dependent acetyl-CoA synthetase 2 (ACSS2) and Terbinafine (squalene epoxidase inhibitor). All inhibitors were purchased at Selleckchemicals (Houston, USA). All inhibitors were aliquoted in DMSO and stored at -20°C.

### Primary cell isolation, culture and characterization

All cell lines were tested regularly for the presence of mycoplasma and have been successfully STR profiled for authentication in April 2021 (LGC, Molsheim, France). Patients (or their guardians) enrolled for therapy at the University Hospital in Bordeaux gave written consent that biological probes can be used for research purposes in an anonymized manner, according to French national guidelines.

A DMG biopsy was received in the laboratory and dissociated using GentleMACs cell separator (Miltenyi Biotech, Paris, France) within 24h using MACSBrain Tumor Dissociation Kit (Miltenyi Biotech, Paris, France) and the standard GentleMACs brain tissue separation program. Cells yielded were cultivated in Amniomax C100SUP plus Amniomax C100 basal medium (Gibco, ThermoFisher, Illkirch Cedex, France). Molecular and cellular analysis of the biopsy are detailed in Figure 5.

For characterization of biopsy-derived cells (BXdmgl) immunohistochemistry was used. Cells were harvested and included in CytoBlocks prior to sectioning and staining (Figure 5A). Automated staining was performed on an Omnis Dako^{®} stainer after heat antigen retrieval in citrate buffer, Flex amplification treatment and incubation with following antibodies: Ki-67 (Dako, clone MIB1, 1/100), H3-K27M (Diagomics, clone RM192, 1/5000) and H3-K27me3 (ABcam, clone 8290, 1/100). Revelation was done using diaminobenzidine (Dako). The cells were regularly passaged and displayed a homogeneous morphological phenotype, they have been termed BXdmgl.

### Western Blots

Cell lysates were prepared by scraping cells using RIPA buffer (Sigma Aldrich) plus proteinase inhibitors (Sigma Aldrich) and centrifuged at 13000g for 15 min at 4°C. Protein concentrations were measured using Pierce^{™} BCATM Protein Assays (ThermoFisher) and equal amounts of cell extracts were loaded for western blot analysis in 4-15 % precasted polyacrylamide gel (Bio-Rad). Proteins were blotted on a nitrocellulose membrane (Transblot^{®} Turbo midi-size, Bio Rad), blocked with the Odyssey blocking buffer (LI-COR Biosciences, ScienceTec, Les Ulis, France) or with BSA diluted in Tris-buffered saline with 0.1% Tween 20 (TBST) and probed with primary antibodies overnight at 4°C. Primary antibodies used were: rabbit polyclonal anti-EZH2 (1:1000 dilution, #5246S, Ozyme/Cells signaling, Saint Cyr l'Ecole, France), rabbit anti-GAPDH (1:15000, BLE649203, Ozyme/Cells signaling), rabbit monoclonal H3K27me3 (1:1000, 9733S, Ozyme/Cells signaling), mouse monoclonal histone H3 (1:500, sc-517576, Santa-Cruz, Heidelberg, Germany), mouse monoclonal actin (C-2) (1:500, sc-8432, Santa-Cruz), diluted in blocking buffer or 5% BSA. After washing with TBST, the membranes were incubated with corresponding goat anti mouse IgG (H+L)-HRP conjugate (1:3000, 170-6516, Biorad, Marnes-la-Coquette, France) or anti-rabbit IgG-HRP (1:3000, A0545, Sigma Aldrich, Saint Quentin Fallavier, France). After washing with TBST (twice for 10 minutes), membranes were revealed with Fusion FX (Vilber Lourmat). Quantification was performed using ImageJ (National Institutes of Health, Bethesda, Maryland, USA).

### siRNA transfections

siRNAs were diluted in the 1x siMAX dilution buffer (30mM HEPES, 100mM KCl, ImM MgCl2, pH=7.3 (Eurofins, Ebersberg, Germany). Cells were transfected independently with each EZH2 siRNA or control siRNA (AllStars Negative Control siRNA, Qiagen, Courtaboeuf, France). Briefly, 10nM of siRNA was transfected with RNAiMAX transfection reagent (Invitrogen) according to the manufacturer's instructions for reverse and forward transfections. Prior to transfection, Lipofectamine RNAiMAX (ThermoFischer) was diluted 1 / 100th in transfection medium (OptiMEM, Gibco, ThermoFisher, Illkirch Cedex, France).

### Cell migration assays and spheroid cultures

Migration was monitored by using the IncuCyte S3 live-cell analysis system (Essen BioScience, Ltd, Royston Hertfordshire, United Kingdom). The original unmodified Incucyte images were used for statistical analysis but for demonstration of the effects images have been transformed using Adobe Photoshop CS4 (San Jose, USA). Following functions have been used to optimize visibility of the cells: greyscale mode, bichrome mode, negative, contrast/luminosity, exposition/gamma, negative.

For spheroid formation assays, 10⁴cells were used per well in a 96-well plate. Volume of methylcellulose/medium/cells and inhibitors (10, 15 or 20 µM) mixture per well was 100 µL with a final concentration of methylcellulose at 0.5%. After rapid and gentle homogenization, the mixture was placed on a round-bottom cell culture microplate (U-shaped) treated to limit cell adhesion. After, spheroids were incubated in an incubator at 37°C and 5% CO2 for 24 hours. Photos and films of spheroids were taken by an InCellis cell imager (Bertin instruments, Montigny-le-Bretonneux, France). Since no dose-dependent effect was observed at the doses tested, all doses were pooled for statistical analysis.

### Chicken and mouse models

For the chick CAM assay DMG model, 1x10⁶ NEM157i/NEM157i-VEGF (50:50) cells were mixed with Matrigel including inhibitors at indicated concentrations and 40 µl were put directly on the CAM. Tumor growth was monitored using a stereomicroscope (DS-Fi2, Nikon/SMZ745T) every two or three days. Tumors were fixed with PFA 4% and proceeded for photo documentation. A similar approach was used for hepatoblastoma Huh6 and HepG2 cells.

For the mouse model, the immortalized SU-DIPG-IV line was used. SU-DIPG-IVi is transduced by a luciferase vector (Luc) with a MOI of 10. Two days after birth, 10⁵ SU-DIPG-IVi-Luc cells in 2 µl were injected directly into the brainstem through the neck to a depth of 3 mm using a 2 µl NeuroSyringe (Hamilton Neuros, Dutscher, Bruxelles, Belgium) under anesthesia with 2% isoflurane and 50% oxygen enrichment. Treatment begun after 8 days, 3-times a week with solvent control (DMSO) or GSK126 (6 or 10 mg/kg), Atorvastatin (10mg/kg) or combo (statin and GSK126 together). Tumor growth was evaluated non-invasively with anesthetized mice on a Biospace imager (Biospace Lab, Nesles la Vallée, France) 1-2 days after each treatment.

Prior to treatments, mice are micro-tattooed (Aramis kit, BiosebLab, France). Animals are weighed and then receive an i.p. injection in the lower right quadrant following IACUC recommendations. A 1 ml tuberculin syringe and a 26-gauge needle were used with an injection volume of 200µl for a 30g mouse (3 mg / ml), adjusted to actual weight each week. Treatment was done 3 times a week, starting at day 8. Tumor growth is evaluated non-invasively on a Biospace imager (Biospace Lab, Nesles la Vallée, France). The entire procedure is carried out on a heating mat at 37°C. The animals are shaved and anesthetized in sterile condition (under PSM-2) in boxes dedicated to imaging. Prior to imaging, animals receive an i.p. injection of 150mg/kg of D-Luc (Promega, E264X) diluted in PBS (50 to 100µl), depending on the weight of the animal. The box is then placed on a heating mat at 37°C in the photo imager in which 2% isoflurane with 50% oxygen is maintained. Imaging is done twice a week. Anesthesia time was around 15 minutes since only immobility is desired and awakening of the animals is almost instantaneous. At day 21, animals were killed by cervical dislocation.

### Statistical methods

Statistical analyzes were performed with GraphPad Prism 5 software (GraphPad Software, Inc. San Diego, USA). For quantitative comparisons of more than two samples, One-way ANOVA test was used followed by Bonferroni post-test. If data distribution was not normal, Kruskal-Wallis test was used following Dunn's post-test of selected relevant conditions. For the comparisons of two small independent samples, unpaired t-test was used. For experiments analyzed by phenotypic evaluation using a semi-quantitative approach, Fisher's Exact test was used. All experiments (except in vivo experiments) were carried out, independently, at least 3 times, n=independent experiments. A p-value of <0.05 was considered to be statistically significant. For all data in figures, *: p< 0.05, **: p< 0.01, ***: p < 0.001 or exact p-values where indicated.

### Example 1.2: Results

### EZH2 gene and protein expression in DMG samples and cell lines

Transcripts of EZH2 are significantly over expressed in DMG samples compared to normal brain (Figure 1A). Similar results have been found in other solid malignancies ranging from prostate, lung, hepatocellular, colorectal, breast to pancreatic cancer. Mean EZH2 over expression was not very elevated compared to controls due to high expression variability between the two groups, but a core set of samples regrouped around the median EZH2 expression and differences are still significant (Figure 1A, left plot, boxed frame). Cell lines used in this study show all expression of EZH2 protein as revealed by Western Blot (Figure 1B).

### DMG cells are sensitive to GSK126 inhibitor

Significant growth inhibition of GSK126 was observed at doses above 6µM and was total at higher doses above 25 µM (Figure 1C). Growth inhibition was accompanied by an increase in tumor cell apoptosis, at similar doses when cell proliferation was strongly reduced (Figure 1D). Moreover, GSK126 efficiently inhibits H3K27me3 trimethylation in DMG cells (Figure 1E). Finally, The half maximal inhibitory concentration (IC50) of GSK126 was measured in three DMG cell lines and ranged from 7.6 to 10.3 µM (Figure 2)

### Effects of ACSS2 inhibitor, Atorvastatin and Terbinafine on DMG cells alone or in combination with GSK126

In order to interpret these changes, we hypothesized that several key enzymes involved in the biosynthesis of cholesterol [squalene epoxidase, hydroxymethylglutaryl-coenzyme A (HMG-CoA) reductase, AcetylCoA acetate-dependent acetyl-CoA synthetase 2] became important to GSK126-treated DMG cells. These enzymes are known for a long time to be implicated in diseases related to lipid metabolisms. For each cholesterol biosynthesis inhibitor tested, no effects have been observed on two different tumor cell lines alone (Figure 3).

However, when co-treated with a low dose of GSK126 (4µM), which does not affect DMG cell growth (Figure 1C, Figure 5D), significant growth inhibition occured for all three inhibitors at doses ranging from 1 to 5µM (Figure 4, A-C).

### Validation of DMG cell line data on freshly isolated DMG cells

Cell lines which are immortalized or cultured for a certain time can behave differently as the original cells. We therefore isolated cells from a DMG biopsy for functional analysis. Bright field microscopy shows a homogenous, spindle-formed cell population (Figure 5A, and insert). H&E staining of BXdmgl cells revealed atypical eosinophilic cells with irregular anisocaryotic cell nuclei with very high mitotic activity, almost all tumor cells express the Ki-67 antigen (Figure 5A). Tumor cells were also strongly positive for the H3K27M mutation and negative for H3K27me3 trimethylation (Figure 5A). Cellular, molecular and genetic characterization of the biopsy from which BXdmgl cells originated, notably confirming the c83A>T; pK28M mutation in histone H3F3A, leading to the driver oncogenic event, which is conserved in BXdmgl cells.

GSK126 treatment reduced H3K27me3 trimethylation in a dose-dependent manner as revealed by Western blots of histone-purified protein extracts (Figure 5A, lower right panel). BXdmgl cells are sensible to growth inhibition by GSK126 (Figure 5B) with an IC50 around 10µM (Figure 5C), comparable to the other DMG lines. BXdmgl cell growth is not affected by three cholesterol biosynthesis inhibitors, up to 10µM (Figure 5D). Combining GSK126 and Atorvastatin showed significant growth inhibition at low doses of 3 and 5µM for each inhibitor, this effect was masked, as expected, at higher doses because of already established cytotoxicity of GSK126 alone (Figure 5E).

### Effects of Atorvastatin, GSK126 and combination on cell migration

We also investigated the influence of the drugs on DMG cell migration using an automated cell scratcher with the Live-Cell IncuCyte^{®} S3 Analysis System (Sartorius). Cell movement was measured from 24h to 48h after confluence and initiation of wound. Initial denuded area is very clean and marked by a frame of the same size for illustration in all conditions (Figure 6A-C). Percent of denuded area covered by migrating DMG cells NEM157i, NEM163i and primary BXdmgl cells after 24h was reported by the Incucyte software and is displayed per condition in the right graphs (Figure 6A-C). For all cells tested, a significant inhibition of migration activity (approximately 2-times) was observed for the combo treatment (p<0.001) compared to single treatments or solvent control. In NEM163i and BXdmgl cells, migration was also slowed by Atorvastatin compared to DMSO (p<0.05 and p<0.001), but cells exposed to combo treatment still had fewer area covered by migrating cells compared to Atorvastatin alone (p<0.001).

### Inhibition of tumor cell spheroid formation by Atorvastatin/GSK126 combination

In an attempt to further investigate cell movements and adhesion phenomena in our cells, we used a protocol which allows spheroid formation of DMG cells and analyzed cell movements and aggregation with an IncuCyte S3 Analysis System. Tumor spheroids are considered as a more realistic culture system than classical 2D models, adding 3D complexity closer to in vivo growth conditions. Using three different DMG cell lines, including our primary BXdmgl cells, we could reliably generate tumor neurospheres with all cell lines (Figures 7A, 7B, 7D). Round-shaped spheres formed rapidly within 24h, albeit with slightly different sizes and sometimes differences in border shape (clear delimitated vs. irregular). However, cells exposed to combo-treatment almost never formed spheroids compared to single GSK126 treatment (p<0.001, for all cells, Figures 7C and 7E). In some GSK126-treated cultures, spheroid formation was affected, especially in the primary cells (Figures 7D, 7E).

### GSK126 effect on orthotopic SU-DIPG-IVi-Luc implanted tumor cells in NOD/LtSz-scid IL2Rgamma (NSG) mice.

We developed an ortothopic brainstem glioma model in newborn immunocompromised mice. SU-DIPG-IVi-Luc cells where generated using lentiviral transfection procedures. In a first experiment, we tested efficacy of GSK126 treatment in this model and found significant growth reduction at an i.p. dose of 10mg/kg evidenced by bioluminescence (p=0.009) in treated mice (n=23) compared to solvent controls (n=18, Figure 8A). For the Atorvastatin/GSK126 combo treatment experiment, we reduced GSK126 dose to 6mg/kg to avoid growth inhibition. After four treatments, a significantly greater tumor growth inhibition of the combo occurred (n=13) compared to Atorvastatin (n=13), GSK126 (n=17) (p<0.05) as well as to DMSO controls (p<0.01, n=13, Figure 8B). No significant differences have been found between controls or single treatments. All bioluminescence images of animals used in this study are shown (Figure 8A, B). Atorvastatin/GSK126 combo treatment also exhibits better anti-tumor effects in a chick CAM DMG model we recently developed. In this short-term model, based on a previously established adult glioma CAM model, drugs can be directly applied on the tumor and growth monitored by biomicroscopy. Phenotypic characterization of drug action can be made by classifying the degree of tumor vascularization into high or low/moderate (Figure 8C, right panel). Degree of vascularization should be interpreted as capacity of tumor cells to interact with the host tissue, an indirect indicator of tumor cell aggressiveness. Combo treated experimental tumors showed reduced vascularization compared to Atorvastatin (p=0.036), GSK126 (p=0.0248) and DMSO controls (p=0.03, Figure 8C, left graph).

### Example 2: Tri-therapies Efficiency for treating and/or preventing hepatoblastoma EZH2 is a central oncogene in proliferative hepatoblastoma

Using classical 2D culture and 3D spheroid models, we found that the depletion of EZH2 by RNA interference blocks the growth of the HB-deriving cell lines Huh6 and HepG2 in vitro by activating senescence (data not shown). In vivo, the depletion of EZH2 totally impeded the development of hepatoblastoma using the chorioallantoic membrane assay (CAM, Indersie et al, Oncotarget, 2017, PMID: 29662633) and reduced tumor cell aggressiveness by lowering tumor angiogenesis (Figure 9). Therefore, EZH2 is a relevant therapeutic target for the treatment of hepatoblastoma.

We then measured the effect of GSK126 and Tazemetostat (also known as EPZ-6438 - see https://pubchem.ncbi.nlm.nih.gov/compound/66558664), two EZH2 inhibitors, on the growth of hepatoblastoma cells. While Tazemetostat was effective at concentrations above 20µM (Figure 10), the IC50 (half maximal effective concentration) of GSK126 was around 6 to 8 µM, a concentration twice lower than that of Cisplatin (Figure 10).

Thus, we tested the effect of combining Cisplatin and GSK126 (see IC50s measured for each drug in each cell line in Figure 10A) to eliminate hepatoblastoma cells using classical 2D culture condition and 3D tumor spheroid. As shown in Figure 11, GSK126 and Cisplatin had an additive effect to kill hepatoblastoma cells in both culture models.

Subsequently, we tested the hypothesis that EZH2 participates in the resistance of hepatoblastoma cells to Cisplatin, a situation often observed in relapsed patients treated with Cisplatin. As shown in Figure 12, the sensitivity of Huh6 and HepG2 cells to Cisplatin was increased by 35 to 48% in absence of EZH2 protein demonstrating the participation of EZH2 in Cisplatin resistance.

As the EZH2 inhibitor GSK126 efficiently kills hepatoblastoma cells and deregulates cholesterol metabolism in DMG cells (see Example 1.2), we tested whether statins synergize with GSK126 and/or Cisplatin to eliminate these tumoral hepatic cells in vitro. As shown in Figure 13A, Simvastatin or Atorvastatin alone had an antitumor effect on Huh6 and HepG2 cells at a concentration of 20µM and 50µM and above, respectively. As expected, the sensitivity of Huh6 and HepG2 cells to GSK126, but not to Cisplatin, was significantly increased (at least twice) in presence of Simvastatin or Atorvastatin (Figure 13A) showing that statins synergize with GSK126 to kill hepatoblastoma cells, but nor with cisplatin. Next, we tested a tri-therapy by combining GSK126, Cisplatin and statins. As shown in Figure 14A-B, the sensitivity of Huh6 and HepG2 cells to the combination of GSK126 + Cisplatin (3µM each) was significantly increased in presence of Simvastatin or Atorvastatin. The tri-therapy GSK126+Cisplatin+statin was significantly more efficient than the combination GSK126+Statin to kill hepatoblastoma cells (Figures 14A-B). Again, Simvastatin was slightly more efficient than Atorvastatin to potentiate the antitumor effect of GSK126 alone or in combination with Cisplatin.

Finally, we tested the hypothesis that the synergistic effect of GSK126 and statins could be extended to other EZH2 methyl-transferase inhibitors. Remarkably, data in Figure 15 show that Simvastatin or Atorvastatin significantly potentiates the sensitivity of hepatoblastoma cells to the EZH2 inhibitor Tazemetostat (E7438/EPZ6438) supporting the idea that statins likely synergize with many, if not all, EZH2 inhibitors.

Altogether, these in vitro and in vivo data in DMG and hepatoblastoma cells strongly suggest that the inhibition of EZH2 enzymatic activity by EZH2 inhibitors (GSK126, Tazemetostat...) activates cholesterol biosynthesis, an adaptive metabolic process which helps tumoral cell to survive and resist to the deleterious effects mediated by EZH2 inhibitors. By combining statins and EZH2 inhibitors, tumoral cells cannot synthesis cholesterol mediated by EZH2 inactivation and are programmed for death through apoptotic processes.

## Claims

1. A composition for use in a method of treating and/or preventing tumors associated with methyltransferase EZH2, comprising administering to a subject in need a therapeutically effective amount of the composition, wherein said composition comprises the combination of an EZH2 inhibitor and one statin, with the proviso that the statin is not lovastatin and wherein the said tumor is not associated with endothelial to mesenchymal transition associated pathology.

2. The composition for use in a method of claim 1 or 2, wherein said EZH2 inhibitors are chosen among GSK126, UNC1999, EPZ005687, Ell, MC3629, GSK926, GSK343, GSK503, CPI-360, CPI-169, CPI-1205, tazemetostat, EPZ011989, ZLD1039, EBI-2511, pinometostat, lirametostat, JQEZ5, PROTAC MS1943, DZNep, MC1947, MC1948, and PF-06821497.

3. The composition for use in a method of claim 1 or 2, wherein tumors associated with methyltransferase EZH2 are selected among hepatoblastoma, Diffuse Intrinsic Pontine Glioma (DIPG), Diffuse Midline Glioma (DMG), bladder cancer, bone cancer, brain cancers, breast cancer, malignant lymphoma, rhabdoid tumor, leukemia, lung cancer, stomach cancer, prostate cancer, colorectal cancer, esophageal cancer, ovarian cancer, uterine cancer, liver cancer, testicular cancer, pancreatic cancer, renal cancer, rectal cancer, thyroid cancer, skin cancer, head & neck cancer.

4. The composition for use in a method of anyone of the preceding claims, wherein said one statin is chosen among atorvastatin, fluvastatin, pitavastatin, pravastatin, rosuvastatin, simvastatin, cerivastatin, and analogs thereof.

5. The composition for use in a method of anyone of the preceding claims, wherein said EZH2 inhibitor is GSK126 or tazemetostat.

6. The composition for use in a method of anyone of the preceding claims, wherein said EZH2 inhibitor is GSK126 or tazemetostat, and wherein said statin is atorvastatin or simvastatin.

7. The composition for use in a method of anyone of the preceding claims, wherein said composition further comprises an effective amount of an anticancer drug.

8. The composition for use in a method of anyone of the preceding claims, wherein said anticancer drug is chosen among platinum compounds, taxane derivatives, topoisomerase inhibitors, hormone therapeutic agents, androgen deprivation agents, androgen receptor agents, serine/threonine kinases inhibitors, tyrosine kinase inhibitors, antiangiogenesis agents, immunotherapeutic preparations, anti-inflammatory drugs, biological preparations having anticancer effects, anticancer preparations made from natural substances.

9. The composition for use in a method of claim 8, wherein said anticancer drug is cisplatin, carboplatin, and/or oxaliplatin.

10. The composition for use in a method of anyone of the preceding claims, wherein said composition is administered to the patient in need via intravenous route, parenteral and non-parenteral routes or local injection.

11. The composition for use in a method of anyone of the preceding claims, wherein said composition is administered to relapsed or refractory patients after anticancer standard treatment, radiotherapy or other first or second line of cancer therapies.
